# EUROPEAN PATENT APPLICATION

(11) **EP 3 854 290 A1**
(43) Date of publication of application: **28.07.2021**
(21) Application number: 20153675.2
(22) Date of filing: 24.01.2020
(51) Int. Cl.: A61B 1/00, A61B 1/06, A61B 1/12, A61B 1/267

(54) **TIP LIGHT LARYNGOSCOPE FOR TRANS-TISSUE ILLUMINATION**

(71) Applicant: Universität Zürich, 8006 Zürich (CH); ETH Zürich, 8092 Zürich (CH)
(72) Inventor: BIRO, Peter, Feldmeilen (CH); GAGE, David, 8800 Thalwil (CH); BOEHLER, Quentin, 8057 Zürich (CH)
(74) Representative: Kleine, Hubertus

(57) **Abstract**

A laryngoscope (1) comprising a handle (7) and a blade (2) with a tip (5) at the end of said blade (2) and a distal light source (6) at said tip (5) to enlighten the environment in front of the tip (5).

## Description

The current invention relates to a light emitting, preferably manually guided, tip light laryngoscope, which light source is built at the tip of the blade to visualize and examine the laryngeal inlet of a patient as a means to assist clinicians inserting the endotracheal tube as it passes through the pharynx towards the bifurcation.

Laryngoscopes are well known to the state of the art as numerous guiding methods, styles, variations and technics for conventional and digital laryngeal intubation have been developed over time.

A laryngoscope is a device which is used by clinicians during tracheal intubation. It assists with intubation by allowing clinicians to visualize the path of the endotracheal tube as it passes the pharynx towards the glottic entrance into the trachea. The pathway can become critical when patients are difficult to intubate, especially in cases of defense reactions due to discomfort, anatomical abnormalities or injuries. This applies in specific when intubation is carried out by less experienced clinicians or such personnel in training.

In most recent developments, a laryngoscope - comprising of a handle and a blade - therefore often includes auxiliary guided means (US2011/0178369 A1) and viewing devices such as cameras and light sources (US9332896B2).

However, where auxiliary means adding to costs of the equipment required to perform efficiently the state of the art still has not reached its possible maximum when it comes to the positioning of the light source within a laryngeal device and the appliance of the light itself in terms of power consumption, wavelength, radiation angle and their characteristics.

It is an object of this invention to seek to mitigate disadvantages such as those described above.

An inventive tip light laryngoscope comprises of a handle and a blade with an end segment and
an adjacent tip. A light source is positioned at said tip to enlighten the tissue in front of this tip while contacting it tightly. The light is provided at this position to perform a trans-tissue illumination, preferably when brought in contact with the tissue in the mucosal fold between tongue base and epiglottis called "vallecula.

There might be a camera installed at said end segment. The camera can be positioned proximal to the tip of the blade towards the perpendicular line of the axis of the handle, wherein the camera is positioned on a side of the blade, which is curved convex, wherein a means of visualization is utilized to view the image from the camera.

The camera can be equipped to capture an image of a part of the environment towards said tip, especially of the end section of the blade. This image could be either a photography or most preferably a video image.

The camera can be a miniaturized version, preferably a charged coupled device (CCD) camera comprising a transmission chip for transmitting images and video images taken from the front view of the intubation blade onto an external display screen. Such screen can be provided as a separate unit outside of the laryngoscope or as attached to the laryngoscope handle.

A detachable, separate display screen might be of advantage when it comes to cleaning and sterilization after use. A regular light emitting light source illuminates the environment in front and around the blade tip.

The image might be visualized and interpreted by the operating clinician or automatically interpreted by an image recognition software that recognizes typical features of the upper airway such as the pharynx, hypopharynx, larynx, glottis, vocal cords, trachea, and bifurcation.

The camera can be manually controlled and operated using provided buttons of the control unit as part of the laryngoscope.

As mentioned above, the laryngoscope comprises a distal light source, also referred to as a tip light, mounted at the tip of the blade to enlighten the tissue in front of the tip. In one preferred example the light source can be actuated but it can also be provided without any external or internal actuation means.

The distal light source can be designed in a way or can be mounted to the tip in a way that the direction of radiation of the distal light source can be set in any direction to best optimize the targeted tissue illumination.

As discussed above, a secondary proximal light source, also which might be preferably a regular light emitting light source, is mounted onto the blade to further enlighten the environment alongside said blade, wherein the secondary proximal light source is preferably positioned proximal from the distal tip.

As it could be understood from the description there could preferebly be at least two light sources for two different purposes integrated in the blade.

The aforementioned secondary proximal light source might be a conventional light source which is inbuilt in every laryngoscope known in the state of the art. It is located towards the end segment of the blade, but not at it's tip. It's position is at about 8-12% of the length of the blade away from the tip.

In the case of a video laryngoscope, where a camera is provided at the blade, it can be placed close to the camera, preferably a CCD camera.

It emits only regular, visible light and it's scope is to illuminate the surroundings - otherwise the eye of the viewer in conventional laryngoscopes or the camera in video laryngoscopes wouldn't see anything.

The light source at the tip of the laryngoscope can preferably be inbuilt into the tip of the blade and has a cylindrical or spherical shape, which is adopted to prevent high pressures on the patient during contact and to avoid contusions. So far such a light source doesn't exist in any of the available laryngoscopes. This light can emit visible light as well as NIR light with a defined wavelength. The light source can be pushed onto the mucosa and get into direct contact with it, preferably in the mucosal fold between tongue base and epiglottis called "vallecula".

The scope of this light is to penetrate the adjacent tissue and to fill it up with the emitted photons in order to produce the "glowing" or "fluorescent effect" of the tissue, especially when NIR is emitted, and viewed via the camera. If visible light is emitted, direct view with the naked eye might be possible too. The light from this source is explicitly NOT meant to illuminate the anatomical features through the air (as does the conventional light source mentioned above).

This formation is designed for better and more secure guidance of the endotracheal tube. A direct light at the front of the moving blade illuminates any tissue in front as well as in the light cone causing fluorescence, particularly to an increased level when the light gets in contact with tissue in way of the moving blade. This visible effect will alert the operating clinician to the extend to either visualize, stop or redirect the movement of the endotracheal tube or to examine the object in question upon its normality or abnormality respectively.

The tip light laryngoscope can also comprise an actuator as part of the light source for example. The purpose of the actuator is to move the illumination angle of the light source

The actuator can be any means of actuation which will be manually controlled and operated via the control unit and its actuating element, which can be a joystick for example. The actuator can also be installed outsight the light source but actuate the light source in order to move the light source in a desired direction.

The tip light laryngoscope can further comprise a control unit which is adjacent to the handle. The control unit can also be an integrated part of the handle. The handle can comprise a power storage to provide electrical power to the camera, the distal light source, the actuator itself and the control unit or any other device, the display screen for example. The power storage can be a compartment or a box which holds any means of power, such as a rechargeable or disposable battery for example.

Optional the devices consisting of the blade, the camera and the distal light source including the actuator can be fitted either stationary or mobile. Alternatively, only the blade can be mobile fitted with an immovable camera and an immovable distal light source and an immovable actuator fixed at defined positions to the blade.

There are multiple advantages for these options. Due to variability in human anatomy, there is no universal size of blade that can be used in all cases. A number of different blades may be desirable and beneficial so that a clinician is able to visualize a laryngeal inlet with a choice of blade shapes and sizes depending on clinical requirements, personal expertise and preference.

The first option - all devices fitted mobile - might be more advantageous in terms of costs in comparison to the second one when it comes to a necessary part-exchange. However, in all cases there is the further advantage when it comes to cleaning and sterilization of the components of the tip light laryngoscope.

Overall this arrangement has multiple advantages. In conjunction with the viewing means as described above, the advantages can be summarized as giving a better controlled guidance of the insertion process of the tracheal tube, providing more security for the patient as injuries of tissue or other parts of the laryngeal inlet potentially caused by a less directly illuminated input path and forward moving blade of a conventional laryngoscope may and can be avoided and ultimately the advantage that this tip light laryngoscope will fit the purpose being a tool for clinicians in training.

In comparison to laryngoscopes of the state of the art the invented tip light laryngoscope might be equipped with such illuminated viewing means and is also cost efficient which adds to the advantages altogether.

Particular embodiments and other and further objects of the invention will be pointed out or indicated in the sub claims hereinafter or will be apparent to one skilled in the art upon an understanding of the invention or implementation of it in practice.

To the best advantage is the embodiment where the camera and the visible light source, also referred to as secondary proximal light source, preferably as regular light emitting light source, are fitted in a back-layered position onto the blade away from the tip, preferably up to 10% of the total length of the blade measured from the tip towards the perpendicular line of the axis of the handle and on the convex curved side of the blade providing an unobstructed view towards the tip.

The distance of the camera lens to the distal edge of the front of the tip can be optimized in a way that it permits the best possible viewing of the pharyngeal structures and the laryngeal opening.

In laryngoscopes according to the state of the art, said tip would possibly touch tissue. Said camera is best positioned backwards from the tip. The tip should be understood the most distal position of blade of the laryngoscope.

An important aspect of this arrangement is that the part of the environment, especially the end section of the blade the image is taken of, includes the tip of the blade, thus the tip will be constantly visible as a point of reference.

By this configuration, the clinician may see and visually confirm the passage of the tracheal tube through the vocal cords into the trachea for example. However the tip light laryngoscope blade does not pass the vocal cords.

The light source at the blade tip can be fitted with a near infrared (NIR) emitting light source or a visible light source or a combination of multiple light sources.

When light hits tissue the effects are in essence reflection, transmittance, dispersion and absorption. In case of absorption, the energy of light will be emitted as heat or fluorescence or phosphorescence. There are a number of absorber in human tissue, H2O is one of them and specifically absorbs NIR.

Depending on the wavelength of the light source, and the consistence of tissue the effects vary. However, using NIR light in medical diagnostic it is known to the art that tissue absorbs NIR light with the effect of fluorescence at wavelengths from 780 nm and at low light power output.

It is therefore advantageous if the light source of the tip light laryngoscope can be fitted with a near infrared emitting light, preferably for the distal light source, with parameters of a wavelength in the region 780 nm to 950 nm, preferably a direction of radiation of 850 nm, and a power consumption below 500 mW, preferably 140 mW maximum.

But it is also possible that the distal light source and/or the secondary proximal light source can also have a wavelength in the visible light region of between 400 nm and 780 nm.

The distal light source and/or the secondary proximal light source can have any combination of visible light and near-infrared light.

A low power consumption is also beneficial as it processes less operating heat.

The light source can be made as a LED or fibreoptic.

It is also of advantage if the distal light source can be fitted centroid at the front of the blade, namely the tip. In this way the light source and the tip form a unit, which can be spherical or cylindrical. Such a position supports and enables a better controllable guidance and avoids the need for any kind of determination of the actual position of the tip of the blade if the light source were fixed differently for example.

The end cap of the blade can be formed spherical or cylindrical. Thus the tip of the blade will have no sharpness of edges which further contributes avoiding potential injuries of tissue for example, when processing the intubation of the blade.

The centroid wavelength can be arrayed in such direction that it forms a normal of the spherical front cap. This array will be of advantage and in line with aforementioned layout of the centroid position of the light source.

The distal light source can be defined in such way that the radiation characteristics are of up to 15° of the direction of radiation of the near infrared emitter light source. This is of advantage as it creates a wider viewing angle around the end of the blade although the detection intensity decreases in relation to the increase of the angle due to the radiation characteristics.

It is therefore advantageous when the light source is either fitted or equipped with an actuator to direct the light cone of the light source using the actuating element of the control unit, such as a joystick, preferably up to at least 45°. Thus the viewing angle can be made broader around the tip of the blade in any direction.

It is of advantage when the blade or the distal light source is fitted with a temperature sensor to configure the operating temperature of the distal light source in a range of 30° C to 40° C, preferably in the range of normal human temperature between 35,7° C and 37,3° C.

The temperature sensor together with a potentially fitted temperature compensation unit that adapts the distal light source operating temperature to an environment, the laryngeal inlet of a human for example, can be regarded as considerable advantage as it makes the usage of such a tip light laryngoscope safe as to the requirements by law and subsequent ordinances and regulations.

The temperature compensation unit can be an integrated part of the control unit or designed as a separate device.

Also the distal light source can be miniaturized with dimensions less than 1,5 mm in diameter and less than 1,75 mm in height.

The blade of the tip light laryngoscope can be shaped in a commonly known way, for example having a thickness double the size next and adjacent to the perpendicular line of the axis of the handle in comparison the one at the tip and preferably having a curved portion with a convex and concave side to smoothly follow and conform to the anatomical shape of a patient's airway.

It is advantageous to have the blade of this tip light laryngoscope shaped in a commonly well-known way, not only due to anatomical reasons but especially when using the tip light laryngoscope for training purposes. A clinician in training will recognize the instrument as such and what it is used for and can instantly concentrate on the specific features applied to it and familiarize with those.

The blade can be manufactured in different materials, for example metals or plastics in which case it could be made opaque or transparent.

The blade along with any attached components including the distal light source can be disconnected from the handle to be cleaned for sanitation or for being disposed.

In a further embodiment the tip light laryngoscope can also comprise a second light source. This second light source can be mounted onto the blade to further enlighten the environment, for example positioned adjacent to the camera.

However, this second light source can also be fitted behind the camera or sidewise of the camera.

An advantageous embodiment of an inventive tip light laryngoscope is further explained in detail by a drawing. Specific parts of the embodiment can be understood as separate features that can also be realized in other embodiments of the invention. The combination of features described by the embodiment shall not be understood as a limitation of the invention:
Fig. 1 a schematic view of the tip light laryngoscope;
Fig. 2 an illustrated view of the light cone and the central direction of radiation;
Fig. 3 an illustrated view of the potential directions of the light cone;
Fig. 4 schematic drawing of an actuated light source;
Fig. 5 position of the laryncoscope in a person showing the effect of illumination due to the position of the light source at the tip of the laryngoscope.

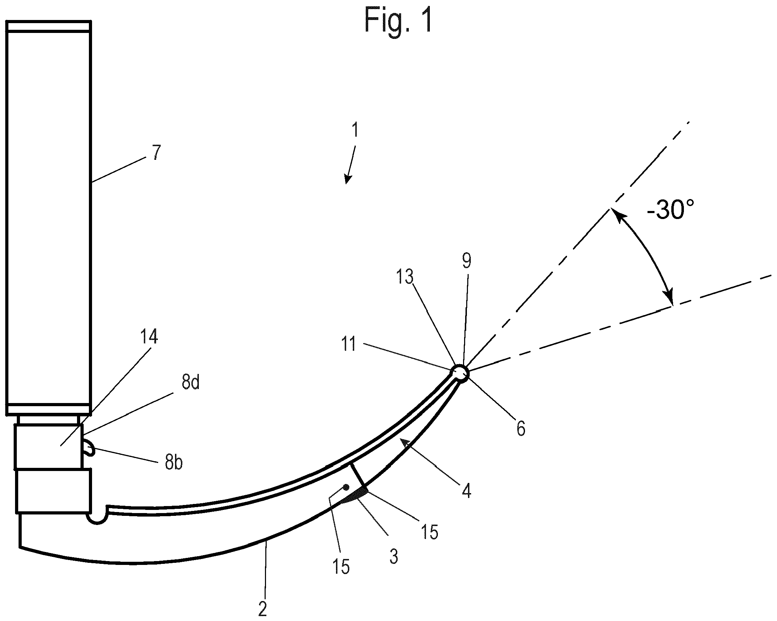

An embodiment of an inventive tip light laryngoscope 1 according to the invention is shown in Fig. 1-3. The tip light laryngoscope 1 comprises a blade 2 which can preferably comprise a camera 3 for taking an image of a part of the environment alongside and in front of an end segment 4 of the blade 2 with a tip 5 and an distal light source 6 at the tip 5 to enlighten the environment in front of the tip 5.

The tip light laryngoscope 1 further comprises a handle 7 and a control unit 8a. The control unit 8a is provided with buttons to operate the camera 3 and the distal light source 6.

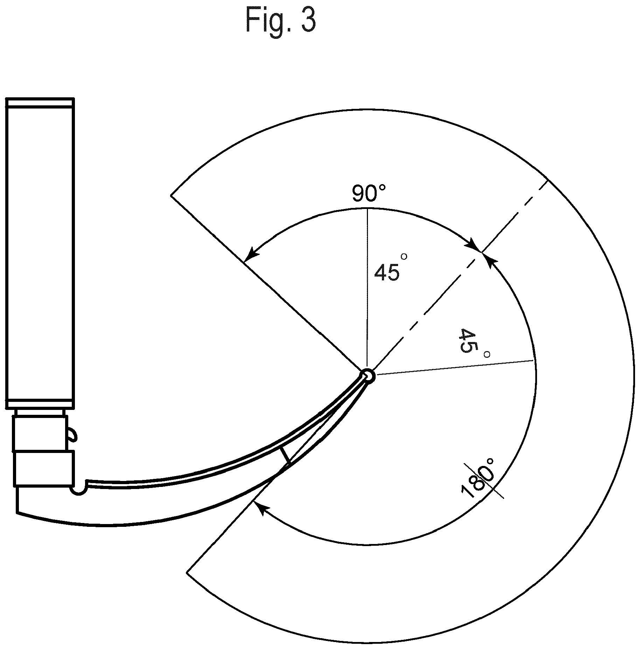

The control unit 8a is further equipped with an actuating element 8b, such as a joystick, to direct the light cone 12 of the distal light source 6 with an equipped actuator 11 in angular directions of preferably up to 45° as illustrated in Fig. 3.

The effect is broadening the viewing angle in contrast to a non-distal light source.

The position of the camera 3 is defined to be up to 10% of the total length of the blade 2 measured from the tip 5 towards the perpendicular line of the axis of the handle 7 and on the convex curved side of the blade 2.

The view of said camera 3 is such that the part of the environment, especially the end segment 4 of the blade 2 an image will be taken of, includes the tip 5 of the blade 2, thus the tip 5 will be constantly visible.

The camera 3 can be a charged coupled device (CCD) camera equipped to take an image of a part of the environment in front of an end segment 4 of the blade 2 with the tip 5 visible, preferably a video image.

The camera 3 can comprise a transmission chip for transmitting images and video images taken from the front view of the intubation blade onto an external display screen.

The control unit 8a can comprise an image recognition software that recognizes typical features of the upper airway by pictures detected by said camera 3.

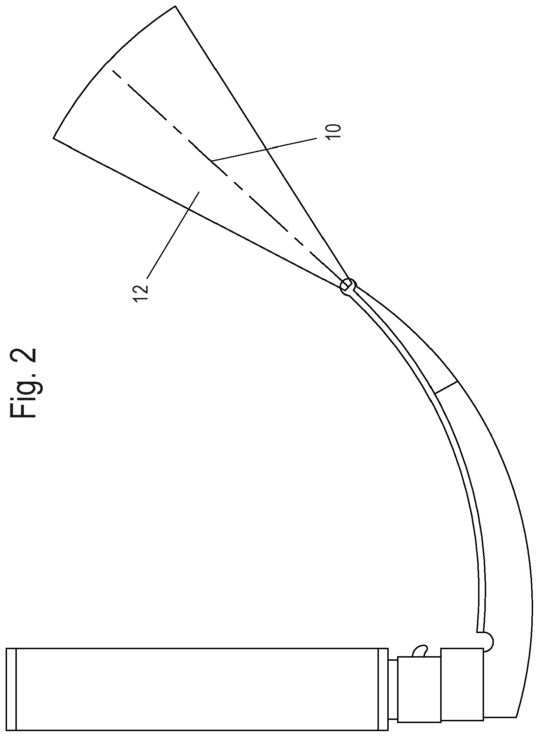

The distal light source 6 is defined as being a near infrared emitter light source. Further defined as being fitted centroid and partly spherical forming a spherical front cap 9 of which a direction of radiation 10 forms a normal. This formation is illustrated in Fig. 2.

The distal light source is further defined being fitted with a near infrared emitter light with a direction of radiation 10 preferably of 850 nm, and a low power consumption such as 140 mW maximum in order to achieve the effect of fluorescence on tissue when operating the tip light laryngoscope 1.

Fig. 1 further shows that in a preferred embodiment the distal light source 6 comprises a temperature sensor 13 to configure it's operating temperature scale, preferably between 35,7°C and 37,3 °C.

Fig. 1 also reveals that the tip light laryngoscope 1 comprises a temperature compensation unit 14 integrated in the control unit 8a that adapts the distal light source 6 operating temperature to an environment, the laryngeal inlet of a human for example.

The temperature sensor 13 and temperature compensation unit 14 are matters of safety.

The blade 2 is defined by a first thickness next and adjacent to the perpendicular line of the axis of the handle 7 and a second thickness at the tip 5 in that way that the first thickness is at least double the size of the second.

The tip light laryngoscope 1 can be fitted mobile in a way that the blade 2 can be detached from the handle 7 in order to apply variations of blades in shape and size.

The blade 2 itself can be mobile fitted with an immovable camera 3 and an immovable distal light source 6 fixed at defined positions to the blade 2.

As an option, the devices consisting of the camera 3 and the distal light source 6 and the actuator 11 can also be fitted mobile.

The tip light laryngoscope can be fitted with a second light source 15 either mounted on top of the camera 3. Alternatively, the second light source 15 can be fitted behind or sidewise of the camera 3.

The tip light 5 in Fig. 1 might be describe as cylindrical in the sense that it is aligned along the tip of the blade. It has an aperture with a limited angle.

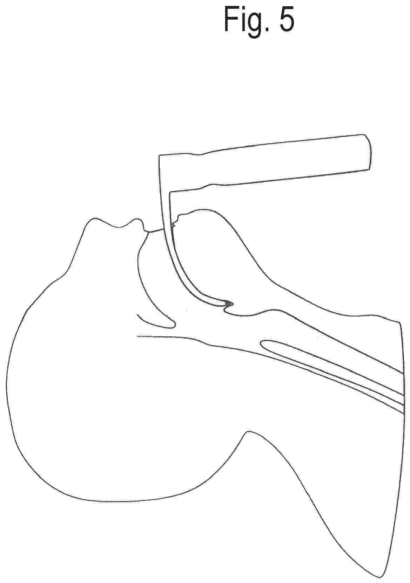

The tip light of Fig. 1 might have a distinct opening angle, which should be up to 35° but not more. The scope of this rather narrow cone is to direct the light more into the tissue as shown in Fig. 5 and to avoid escaping into the surroundings.

The direction of the tip light beam might be as a continuation of the blade, or in a specific angle downwards see Fig. 1 e.g. by 30°.

The tip light might be equipped with LED units with different wavelengths and the actual combination of emitted light wave length may be variated by the user, either by setting changes or by a dedicated switch. The same might be true for the light intensity.

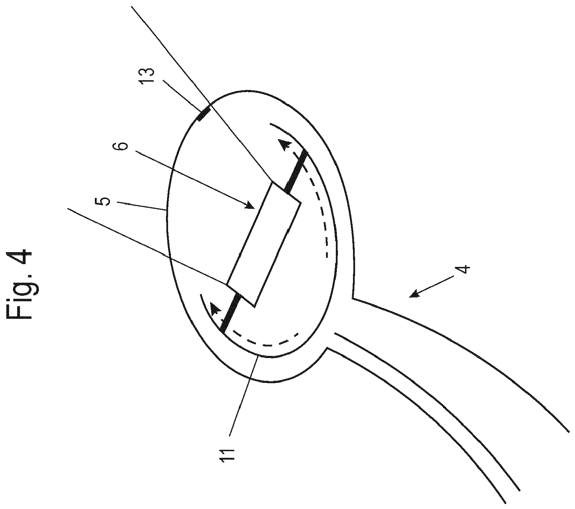

Fig. 4 discloses an example of an actuated light source 6, where the light cone can be moved by an actuator such as a partly spherical actuator which can be moved inside a transparent housing in the same manner as a ball-joint. The light source 6, such as an LED, can be connected to this actuator 11 and thus the light cone of the light source can be moved in any suitable direction. The housing can be further provided with the said temperature sensor 13 at a position where the light will be guided through the housing, thus heating the housing to a certain amount.

In a further embodiment, the tip light laryngoscope can be provided with a contact sensor, wherein the tip light is switched on only when the tip is in contact with a tissue, which is the case in Fig. 5. It might be also possible that the tip light can be switched on by a switch at the handle or near the handle of said laryngoscope. For an orientation the environmental light provided by the second light source, for example at the camera, can be always switched on.

Most preferably the control of the first and the second light source can be done independently from each other as described by the aforementioned examples.

One further aspect of the current invention is a method for performing guided tracheal intubation in preparation and/or during the introduction of an endotracheal tube on a subject with an inventive tip light laryngoscope.

Said method comprises the step of internally illuminating the tissue of the subject in the region of the subject's vallecula with the light source at the tip of said laryngoscope which is brought in contact with said tissue, where the illumination is enhanced by the anatomical structure of the subject in this region. This step is provided to facilitate the guiding of an endotracheal tube during its introduction.

### Reference numeral

- 1: laryngoscope
- 2: blade
- 3: camera
- 4: end segment
- 5: distal tip
- 6: distal light source
- 7: handle
- 8a: control unit
- 8b: actuating element
- 9: spherical front cap
- 10: direction of radiation
- 11: actuator
- 12: light cone
- 13: temperature sensor
- 14: temperature compensation unit
- 15: secondary proximal light source
- 16: trans-tissue illumination

## Claims

1. A tip light laryngoscope (1) adapted for the trans-tissue illumination during insertion of the laryngoscope, preferably in the mucosal fold between tongue base and vallecula, comprising a handle (7) and a blade (2) with a tip (5) at the end of said blade (2) and a distal light source (6) at said tip (5) to enlighten the environment in front of the tip (5).

2. The tip light laryngoscope (1) according to claim 1, **characterized in that** a camera (3) is positioned proximal to the tip (5) of the blade (2) towards the perpendicular line of the axis of the handle (7), wherein the camera (3) is positioned on a side of the blade (2) which is curved convex, wherein a means of visualization is utilized to view the image from the camera (3).

3. The tip light laryngoscope (1) according to claim 1 or 2, **characterized in that** the direction of radiation (10) of the distal light source (6) can be set in any direction to best optimize the targeted tissue illumination (16).

4. The tip light laryngoscope (1) according to one of the preceding claims, **characterized in that** a secondary proximal light source (15) is mounted onto the blade (2) to further enlighten the environment alongside said blade, wherein the secondary proximal light source (15) is preferably positioned proximal from the distal tip (5).

5. The tip light laryngoscope (1) according to any preceding claim, **characterized in that** the distal light source (6) and/or the secondary proximal light source (15) has a wavelength in the near-infrared region of between 780 nm and 950 nm.

6. The tip light laryngoscope (1) according to any preceding claim, **characterized in that** the distal light source (6) and/or the secondary proximal light source (15) has a wavelength in the visible light region of between 400 nm and 780 nm. The tip light laryngoscope (1) according to any preceding claim, **characterized in that** the blade (2) along with any attached components including the distal light source (6) can be disconnected from the handle (7) to be cleaned for sanitation or for being disposed.

7. The tip light laryngoscope (1) according to one of the preceding claims, **characterized in that** the distal light source (6) is fitted and/or equipped with an actuator (11) which is controllable by a control unit (8a), preferably by an actuating element (8b) of said control unit (8a), to direct the light cone (12) of the distal light source (6).

8. The tip light laryngoscope (1) according to one of the preceding claims, **characterized in that** the blade (2), preferably the distal light source (6), comprises a temperature sensor (13) to configure the operating temperature scale of said distal light source (6) between 30°C and 40°C, preferably between 35,7°C and 37,3 °C.

9. The tip light laryngoscope (1) according to one of the preceding claims, **characterized in that** the laryngoscope (1) comprises a temperature compensation unit (14) to adapt its operating temperature to an environment.

10. The tip light laryngoscope (1) according to one of the preceding claims, **characterized in that** the distal light source (6) has a diameter less than 1,5 mm and a height less than 1,75 mm.

11. The tip light laryngoscope (1) according to one of the preceding claims, **characterized in that** the light source (6) is provided with an an aperture with a limited opening angle, preferably less or equal to 35°.

12. The tip light laryngoscope (1) according to one of the preceding claims, **characterized in that** the direction of the tip light beam emitted by said light source (6) might be as a continuation of the blade (2), or in a specific angle bend only in one direction from said blade.

13. The tip light laryngoscope (1) according to one of the preceding claims, **characterized in that** the light source (6) are equipped with LED units with different wavelengths and/or light intensities such that the actual combination of emitted light wavelength and intensity can be variated by the user, either by setting changes and/or by a dedicated switch.

14. A method for performing guided tracheal intubation for the introduction of an endotracheal tube on a subject with a tip light laryngoscope (1) according to one of the preceding claims, comprising;
internally illuminating the tissue of the subject in the region of the vallecula with the light source (6) at the tip (5) of said laryngoscope (1) which is brought in contact with said tissue in the mucosal fold, where the illumination is enhanced by the anatomical structure of the subject in this region and hence facilitating the guiding of an endotracheal tube.
